# EUROPÄISCHE PATENTSCHRIFT

(11) **EP 0 411 735 B1**
(45) Veröffentlichungstag und Bekanntmachung des Hinweises auf die Patenterteilung: **17.05.1995**
(21) Anmeldenummer: 90250201.2
(22) Anmeldetag: 06.08.1990
(51) Int. Cl.: C07D 233/58, C07D 249/08, C07D 249/04, C07D 409/06, A61K 31/41

(54) **Cycloakylenazole, Verfahren zu deren Herstellung, pharmazeutische Präparate, die diese enthalten sowie ihre Verwendung zur Herstellung von Arzneimitteln**
Cycloalkylenazoles, process for their preparation, pharmaceutical preparations containing them and their use for the preparation of medicines
Cycloalkylène-azoles, leur procédé de préparation, formes pharmaceutiques les contenant ainsi que leur application à la préparation de médicaments

(30) Priorität: 04.08.1989 DE 3926365
(43) Veröffentlichungstag der Anmeldung: 06.02.1991
(73) Patentinhaber: SCHERING AKTIENGESELLSCHAFT, 13342 Berlin (DE)
(72) Erfinder: Bohlmann, Rolf, Dr., D-1000 Berlin 19 (DE); Strehlke, Peter, Dr., D-1000 Berlin 12 (DE); Henderson, David, Dr., D-1000 Berlin 28 (DE); Schneider, Martin, Dr., D-1000 Berlin 12 (DE); Nishino, Yukishige, Dr., D-1000 Berlin 22 (DE)

(56) Entgegenhaltungen:
- EP-A- 0 227 100
- EP-A- 0 236 940

## Beschreibung

Die Erfindung betrifft Cycloalkylenazole der allgemeinen Formel I 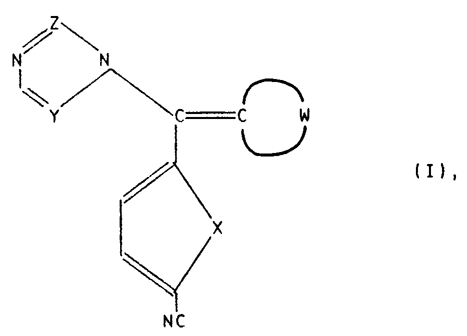
worin 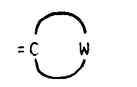
für ein carbocyclisches oder carbopolycyclisches System, das gegebenenfalls mindestens einen Alkylsubstituenten trägt,
X für die Gruppierung 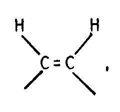
für ein Sauerstoff- oder Schwefelatom und
Y und Z unabhängig voneinander für eine Methingruppe 〉̶CH oder ein Stickstoffatom
stehen,
sowie deren pharmazeutisch verträgliche Additionssalze mit Säuren, ein Verfahren zur Herstellung der Cycloalkylenazole der allgemeinen Formel I, pharmazeutische Präparate die diese Cycloalkylenazole enthalten sowie ihre Verwendung zur Herstellung von Arzneimitteln. 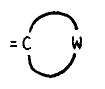
bedeutet dabei vorzugsweise einen Cyclopentyliden-, Cyclohexyliden-, Cycloheptyliden- oder Adamantylidenrest. Auch ein Cyclobutyliden-, Cyclooctyliden-, Cyclononyliden-, Cyclodecyliden-, Bicyclo[3,3,1]-non-9-yliden-oder Spiro-[5,5]undec-3-yliden-rest kommt bevorzugt infrage.
Wenn der Rest 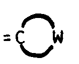
Alkylsubstituenten trägt, sind dies in erster Linie ein oder mehrere geradkettige oder verzweigte C₁- bis C₆-Alkylreste, insbesondere 1 bis 4 geradkettige oder verzweigte C₁- bis C₄-Alkylreste.

Für X steht vorzugsweise eine 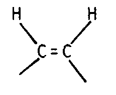
-Brücke oder ein Schwefelatom.

Y und Z werden vor allem so gewählt, daß ein 1-Imidazolyl-, 1,2,3-Triazolyl- oder 1,2,4-Triazolylring zustande kommt.
Als Salze der Cycloalkylenazole der allgemeinen Formel I sind insbesondere die physiologisch und pharmakologisch verträglichen Salze mit der Malonsäure, der Bernsteinsäure, mit Chlorwasserstoff und Bromwasserstoff zu nennen.

Nachstehende Verbindungen sind erfindungsgemäß besonders bevorzugt:
4-[1-Cyclohexyliden-1-(imidazolyl)-methyl]-benzonitril, Hydrochlorid;
4-[1-Cyclopentyliden-1-(imidazolyl)-methyl]-benzonitril;
4-[1-Cycloheptyliden-1-(imidazolyl)-methyl]-benzonitril;
4-[2-Adamantyliden-1-(imidazolyl)-methyl]-benzonitril;
4-[1-Cyclohexyliden-1-(1,2,4-triazolyl)-methyl]-benzonitril;
4-[1-Cyclopentyliden-1-(1,2,4-triazolyl)-methyl]-benzonitril;
4-[1-Cycloheptyliden-1-(1,2,4-triazolyl)-methyl]-benzonitril;
4-[2-Adamantyliden-1-(1,2,4-triazolyl)-methyl]-benzonitril;
4-[1-Cyclohexyliden-1-(1,2,3-triazolyl)-methyl]-benzonitril;
4-[1-Cyclopentyliden-1-(1,2,3-triazolyl)-methyl]-benzonitril;
5-(Cyclohexyliden-1-imidazolylmethyl]-thiophen-2-carbonitril.
4[1-(3,3,5,5-Tetramethyl-1-cyclohexyliden)-1-(1-imidazolyl)-methyl]-benzonitril;
4-[1-Cycloheptyliden-1-(1,2,3-triazol-1-yl)-methyl]-benzonitril;
4-[1-Cyclodecyliden-1-(1-imidazolyl)-methyl]-benzonitril;
4-[1-Cycloheptyliden-1-(1-imidazolyl)-methyl]-benzonitril, Hydrochlorid;
4-[1-(4-tert.-Butyl-1-cyclohexyliden)-1-(1-imidazolyl)-methyl]-benzonitril;
4-[1-(3,4-Dimethyl-1-cyclopentyliden)-1-(1-imidazolyl)-methyl]-benzonitril;
(Isomerengemisch cis (trans);
4-[1-(3,5-Dimethyl-1-cyclohexyliden)-1-(1-imidazolyl)-methyl]-benzonitril;
4-[1-(4-Methyl-1-cyclohexyliden)-1-(1-imidazolyl)-methyl]-benzonitril;
4-[1-Cyclononyliden)-1-(1-imidazolyl)-methyl]-benzonitril;
4-[1-(Bicyclo[3,3,1]-non-9-yliden)-1-(1-imidazolyl)-methyl]-benzonitril;
4-[1-(1-Imidazolyl)-1-(spiro[5,5]undec-3-yliden)-methyl]-benzonitril;
4-[1-Cyclooctyliden)-1-(1-imidazolyl)-methyl]-benzonitril;
4-[1-(2,6-Dimethyl-1-cyclohexyliden)-1-(1-imidazolyl)-methyl]-benzonitril;
4-[1-Cyclohexyliden-1-(1-imidazolyl)-methyl]-benzonitril;
4-[1-(2-Adamantyliden)-1-(1,2,3-triazol-1-yl)-methyl]-benzonitril;
4-[1-(2-Adamantyliden)-1-(1,2,4-triazol-1-yl)-methyl]-benzonitril;
4-[1-(2-Adamantyliden)-1-(1-imidazolyl)-methyl]-benzonitril, Hydrochlorid;
4-[1-Cyclobutyliden)-1-(1-imidazolyl)-methyl]-benzonitril;
5-[Cyclopentyliden-(1-imidazolyl)-methyl]-thiophen-2-carbonitril
Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimmulierten Tumoren, wie zum Beispiel Mammakarzinom oder Prostatahyperplasie (The Lancet, 1984, 1237-1239).

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.
Ferner können die Verbindungen bei Frauen im fortpflanzungsfähigem Alter als Antifertilitätsmittel verwendet werden, um Ovolationen durch Östrogenentzug zu hemmen.

Wahrscheinlich sind Aromatasehemmer auch zur Behandlung des drohenden Herzinfarktes geeignet, da erhöhte Östrogenspiegel beim Mann einem Herzinfarkt vorangehen können (US-Patent 4,289,762).
Bekannte, eine aromatasehemmende Wirkung aufweisende Substanzen sind neben Steroiden auch nichtsteroidale Substanzen, beispielsweise die in den europäischen Patentanmeldungen, EP-A 0165777 bis 0165784 beschriebenen diversen Stickstoffheterocyclen, die in J. Med. Chem. 1986, 29. Seiten 1362-1369 beschriebenen substituierten Glutarsäureimide, die in der europäischen Patentanmeldung EP-A 0165904 beschriebenen substituierten Imidazobenzole, die in der europäischen Patentanmeldung EP-A 0236940 beschriebenen substituierten heterocyclisch substituierten Toluolnitrile sowie die aus dem US-Patent US-A-4,728,465 hervorgehenden, einen gegebenenfalls substituierten Phenylring tragenden Imidazo- und 5,6,7,8-Tetrahydroimidazo[1,5a]pyridine, aus denen insbesondere das 5-(p-Cyanophenyl)-5,6,7,8-tetrahydroimidazo[1,5a]pyridin, Hydrochlorid als stark wirksamer Aromatasehemmer herausragt (Cancer Res. 48, S. 834-838, 1988).

Die Verbindungen der vorliegenden Anmeldung zeichnen sich gegenüber den bisher bekannten Verbindungen dadurch aus, daß sie das Enzymsystem der Aromatase stärker und zugleich selektiver hemmen. Die selektive Wirkung zeigt sich daran, daß andere Enzymsysteme in geringerem Umfang beeinträchtigt werden.
Weiterhin zeichnen sich die Verbindungen der vorliegenden Anmeldung dadurch aus, daß sie nicht chinal sind und somit nicht als 1:1 Mischung der unterschiedlich wirkenden Antipoden auftreten können.

Die aromatasehemmende Wirkung der erfindungsgemäßen Substanzen läßt sich sowohl in-vitro (Test a)) als auch in-vivo (Test b)) zeigen. Folgende Substanzen werden stellvertretend untersucht:

### Test a)

4-[1-Cyclohexyliden-1-(1-imidazolyl)-methyl]-benzonitril, Hydrochlorid (A)
4-[1-Cyclohexyliden-1-(1-imidazolyl)-methyl]-benzonitril (B),

### Test b)

Verbindung A,
Verbindung B,
4-[1-Cyclopentyliden-1-(1-imidazolyl)-methyl]-benzonitril (C);
Als Vergleichssubstanz in beiden Tests dient der aus der US-A-4,728,645 bekannte Aromatasehemmer 5-(p-Cyanophenyl)-5,6,7,8-tetrahydroimidazo[1,5a]pyridin, Hydrochlorid in Form des Racemats (D).

### Beschreibung Test a) (Aromatasehemmung in menschlicher Placenta)

Die Fähigkeiten der Verbindungen, das Enzymsystem der Aromatase zu inhibieren, wird an Mikrosomen, erhalten aus Humanplacenta, getestet. Die Freisetzung von Tritium-markiertem Wasser (³H₂O), das als Reaktionsprodukt bei der Aromatisierung des [1β-³H]- Andostrendions zum Östron freigesetzt wird, wird nach der Methode von Thompson und Siiteri (J. Biol. Chem., 249, 5364-72 (1974)) gemessen. Die entsprechenden Hemmwerte (Kᵢ, Aromatase) werden durch graphische Bestimmung, der Auftragung von 1/v gegen die Hemmkonzentration, gemäß der Methode von Dixon (Biochem. J 94, 760 (1965) bestimmt.

| Verbindung | Ki, Aromatase |
|---|---|
| 4-OH-Androstendion* | 62,0 nmol/l |
| A | 0,19 nmol/l |
| D | 1,2 nmol/l |
| 4-OH-Androstendion* | 41,0 nmol/l |
| B | 0,48 nmol/l |

| | |
|---|---|
| *) als Bezugssubstanz | |

### Beschreibung Test b) (Beeinflussung des durch Androstendion induzierten Ute ruswachstums bei infantilen Ratten)

Infantile weibliche Ratten erhalten täglich einmal über drei Tage die zu testende Substanz in Kombination mit Androstendion. Eine Gruppe bekommt nur Androstendion und eine weitere nur das Vehikel. Einen Tag nach der letzten Behandlung werden die Tiere durch Dekapitation und Entblutung getötet und die Frischgewichte der Uteri (ohne Inhalt) ermittelt. Die Uterusgewichte werden auf mg/100g Körpergewicht umgerechnet. Für jede Gruppe werden der Mittelwert und die Standardabweichung errechnet. Die Signifikanzen der Unterschiede zur Kontrollgruppe (Androstendiongruppe bzw. Vehikelgruppe) werden durch Varianzanalyse geprüft. Es wird außerdem die prozentuale Hemmung des Uteruswachstums bezogen auf die Androstendiongruppe ermittelt. Dabei wird das Uterusgewicht der Vehikelgruppe (Basiswert) von dem entsprechenden Gewicht der Androstendiongruppe abgezogen. Auf den so erhaltenen Wert werden die einzelnen Dosierungsgruppen nach Abzug des Basiswertes bezogen.

| Verbindung | Dosis mg/kg/Tag | Uterusgewicht mg/100g KG* | Hemmung in % |
|---|---|---|---|
| Kontrolle | Vehikel# | 61,0 ± 16,1 | |
| Androstendion (AD) | 30 | 213,6 ± 32,4 | |
| A + AD | 2,0 + 30 | 100,1 ± 18,8 | 74 |
| A + AD | 0,2 + 30 | 108,2 ± 11,9 | 69 |
| D + AD | 0,2 + 30 | 83,2 ± 6,9 | 86 |
| Kontrolle | Vehikel | 48,8 ± 5,0 | |
| Androstendion (AD) | 30 | 183,5 ± 22,7 | |
| B + AD | 2,0 + 30 | 72,5 ± 10,3 | 82 |
| C + AD | 2,0 + 30 | 69,2 ± 5,7 | 85 |
| D + AD | 0,2 + 30 | 69,9 ± 6,6 | 84 |

| | | | |
|---|---|---|---|
| *KG = Körpergewicht | | | |
| # Myrj^{(R)} + phys. Kochsalzlösung (1:1) | | | |

Im Vergleich zu den strukturell sehr nahe stehenden Verbindungen der EP-A 0236940 weisen die erfindungsgemäßen Verbindungen der allgemeinen Formel I am Kohlenstoffatom, an dem sich sowohl der Cyanoaryl- als auch der N-Heteroarylrest befindet, durch die Einführung der Doppelbindung kein Chiralitätszentrum mehr auf. Durch die Aufhebung des Chiralitätszentrums werden Probleme bei der Aufarbeitung und Trennung steroisomerer Verbindungen vermieden.
Die zu verabreichende Menge der Verbindungen schwankt innerhalb eines weiten Bereichs und kann jede wirksame Menge abdecken. In Abhängigkeit des zu behandelnden Zustands und der Art der Verabreichung kann die Menge der verabreichten Verbindungen 0,0001-10 mg/kg Körpergewicht, vorzugsweise 0,001-1 mg/kg Körpergewicht, je Tag betragen.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw., enthalten. Die einzelnen Dosierungseinheiten für die orale Applikation können beispielsweise 0,05-50 mg des Wirkstoffes (Aromataseinhibitors) enthalten.

Zur parenteralen Verabreichung können die Wirkstoffe in einem physiologisch verträglichen Verdünnungmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiergemisches verwendet. Als Beispiele für verwendete Öle sind zu nennen; Olivenöl, Erdnußöl, Baumwollöl, Sojabohnenöl, Rizinusöl und Sesamöl.

Die Verbindungen lassen sich auch in Form einer Depotinjektion oder eines Implantatpräparates anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silicone, wie zum Beispiel Siliconkautschuk. Die Wirkstoffe können außerdem zur perkutanen Aplikation zum Beispiel in Pflaster eingearbeitet werden.

Die Erfindung betrifft somit auch pharmazeutische Präparate und die Verwendung der Verbindungen zur Herstellung dieser Präparate zur Behandlung von östrogenbedingten Krankheiten.

Die Erfindung betrifft ferner ein Verfahren zur Herstellung von Cycloalkylenazolen der allgemeinen Formel I.
Dabei wird eine Verbindung der allgemeinen Formel II 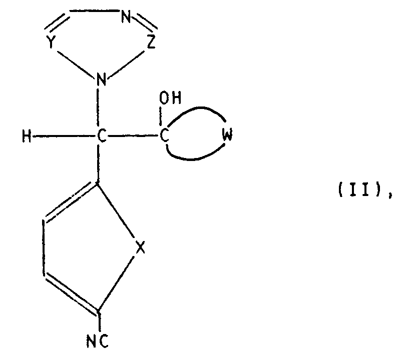
worin 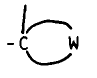
für ein carbocyclisches oder carbopolycyclisches System steht sowie X, Y und Z die in Formel I angegebene Bedeutung haben,
gegebenenfalls nach Überführung der Hydroxygruppe in eine bessere Abgangsgruppe A, entweder durch Erhitzen oder durch Zugabe einer Base, gegebenenfalls unter zusätzlichem Erhitzen durch Abspaltung von HOH oder, falls die Hydroxygruppe in eine Abgangsgruppe A überführt wurde, durch Abspaltung von HA oder
daß ein Dihalogenid der allgemeinen Formel III 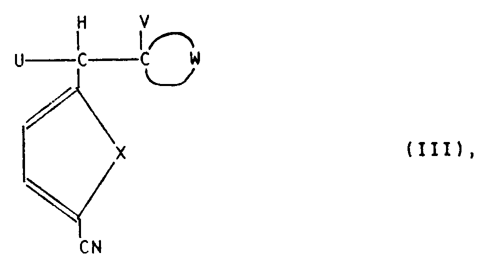
worin 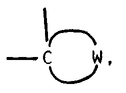
X, Y und Z die in Formel I angegebene Bedeutung haben und U und V Halogenatome bedeuten, die gleich oder verschieden, vorzugsweise 2 Bromatome, sein können, durch Umsetzung mit einem Heterocyclus der allgemeinen Formel IV 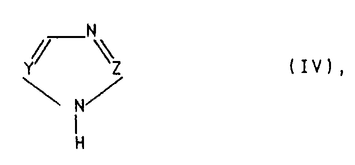
worin
Y und Z die in Formel I angegebene Bedeutung haben, gegebenenfalls unter Verwendung einer Hilfsbase, in eine Verbindung der allgemeinen Formel I und diese gegebenenfalls durch Umsetzung mit Säure in ein pharmazeutisch verträgliches Additionssalz überführt wird.
Bei der ersten Variante wird vorzugsweise die Hydroxygruppe vor der Einführung der Doppelbindung in eine Chlorfunktion durch Umsetzung der Hydroxyverbindung beispielsweise mit Thionylchlorid überführt. Die anschließende Abspaltung von Chlorwasserstoff kann dann einfach durch weiteres Erhitzen im Chlorierungsmittel bewerkstelligt werden. Im allgemeinen ist es jedoch zweckmäßig, die Einführung der Doppelbindung durch Abspaltung von HOH oder HA unter Einwirkung einer Base vorzunehmen. Als Base kommen neben anderen beispielsweise mäßig konzentrierte wäßrige Natronlauge oder Triethylamin infrage.

Bei der zweiten Variante erfolgt die Umsetzung einer Verbindung der Formel III mit einer Verbindung der Formel IV mit oder ohne Lösemittel, wobei im letzteren Fall die Temperatur zwischen dem Schmelzpunkt der Verbindung IV und 200°C, vorzugsweise zwischen 120° und 170°C liegt. Als Lösemittel kommen beispielsweise Acetonitril, Dimethylformamid, Benzol, Toluol oder Chlorbenzol infrage.
Hilfsbasen können tertiäre Amine wie Triethylamin, Tributylamin, Dimethylanilin oder Pyridin sein aber auch Alkalimetallhydride wie Natriumhydrid, Kaliumhydrid oder Lithiumhydrid oder Alkalialkoholate wie Kalium-tert.-butanolat, die aus der Verbindung der Formel IV ein Alkalisalz erzeugen.

Überführung der freien Basen der allgemeinen Formel I in die erfindungsgemäßen pharmakologisch verträglichen Säureadditionssalze erfolgt durch Umsetzung der Base mit der entsprechenden Säure nach Standardvorschriften.

Weitere Einzelheiten zur Herstellung der Verbindungen der allgemeinen Formel I ergeben sich aus den nachfolgenden Synthesebeispielen 1-11, aus denen auch die Darstellung der jeweiligen Ausgangsverbindung der allgemeinen Formel II zu entnehmen ist. Weitere Ausgangsverbindungen der allgemeinen Formel II lassen sich durch analoges Vorgehen unter Verwendung der entsprechend angepaßten Reaktionspartner herstellen.

### Beispiel 1

### 4-[1-Cyclohexyliden-1-(imidazolyl)-methyl]-benzonitril,Hydrochlorid.

### a) 4-[1-(Imidazolyl)-methyl]-benzonitril.

23,15 g Imidazol werden in 250 ml N,N-Dimetylformamid unter Kühlung portionsweise mit 14,8 g Natriumhydrid 60% in Öl versetzt und 1,5 h bei 25° bis zum Ende der Wasserstoffentwicklung gerührt. Zu dieser Lösung werden 50 g 4-(Brommethyl)-benzonitril in 2 Portionen unter Eiskühlung zugegeben und 1,5 h bei 25° gerührt. Zur Aufarbeitung werden unter Eiskühlung 100 ml Wasser zugesetz, 0,25 h gerührt, in 1,5 l Essigester gegossen, mit Wasser neutral gewaschen, zweimal mit Kochsalz und Wasser gewaschen, über Natriumsulfat getrocknet, im Vakuum zur Trockne eingeengt und aus Aceton/Diethylether kristallisiert. Man erhält 33,6 g 4-[1-(Imidazolyl)-methyl]-benzonitril als farblose Kristalle vom Schmelzpunkt 102°.

### b) 4-[1-Cyclohexyliden-1-(imidazolyl)-methyl]-benzonitril,Hydrochlorid.

1,83 g 4-[1-(Imidazolyl)-methyl]-benzonitril werden in 50 ml Tetrahydrofuran gelöst und bei -50° mit 7,3 ml 1,5 molarer Lösung von Lithiumdiisopropylamid in Tetrahydrofuran versetzt, 0,5 h gerührt, mit 1,05 g Cyclohexanon 1 h bei - 60° nachgerührt und auf 25° erwärmt Dann wird Wasser zugegeben, mit Essigester verdünnt, mit Wasser neutralgewaschen, über Natriumsulfat getrocknet und im Vakuum zur Trockne eingeengt. Man erhält 2,7 g rohes 4-[1-Hydroxycyclohex-1-yl-1-(imidazolyl)-methyl]-benzonitril.
1,8 g rohes 4-[1-Hydroxycyclohex-1-yl-1-(imidazolyl)-methyl]-benzonitril werden in 20 ml Thionylchlorid 1 h rückfluxiert. Dann wird im Vakuum zur Trockne eingeengt und an Kieselgel mit Dichlormethan/Methanol chromatographiert. Man erhält 304 mg der freien Base vom Schmelzpunkt 128°, die mit Salzsäure ins ölige 4-[1-Cyclohexyliden-1-(imidazolyl)-methyl]-benzonitril, Hydrochlorid überführt wird.

### Beispiel 2

### 4-[1-Cyclopentyliden-1-(imidazolyl)-methyl]-benzonitril.

5.0 g 4-[1-(Imidazolyl)-methyl]-benzonitril werden in 150 ml Tetrahydrofuran gelöst und bei -50° mit 20 ml 1,5 molarer Lösung von Lithiumdiisopropylamid in Tetrahydrofuran versetzt, 0,5 h bei -60° gerührt, mit 2,5 g Cyclopentanon versetzt, 1 h nachgerührt und auf 25° erwärmt. Dann wird Wasser zugegeben, mit Essigester verdünnt, mit Wasser neutralgewaschen, über Natriumsulfat getrocknet und im Vakuum zur Trockne eingeengt. Man erhält 6,9 g rohes 4-[1-Hydroxycyclopent-1-yl-1-(imidazolyl)-methyl]-benzonitril.
6,9 g rohes 4-[1-Hydroxycyclopent-1-yl-1-(imidazolyl)-methyl]-benzonitril werden in 82 ml Thionylchlorid 0,5 h rückfluxiert Dann wird im Vakuum zur Trockne eingeengt, mit 2 normaler Natronlauge versetzt mit Dichlormethan extrahiert, über Natriumsulfat getrocknet, im Vakuum zur Trockne eingeengt und an Kieselgel mit Hexan/Essigester chromatographiert Man erhält 3,1 g 4-[1-Cyclopentyliden-1-(imidazolyl)-methyl]-benzonitril als freie Base vom Schmelzpunkt 108-110°.

### Beispiel 3

### 4-[1-Cycloheptyliden-1-(imidazolyl)-methyl]-benzonitril.

10 g 4-[1-(Imidazolyl)-methyl]-benzonitril werden in 270 ml Tetrahydrofuran gelöst und bei -50° mit 40 ml 1,5 molarer Lösung von Lithiumdiisopropylamid in Tetrahydrofuran versetzt, 0,5 h bei -60° gerührt, mit 6,5 g Cycloheptanon versetzt. 1 h nachgerührt und auf 25° erwärmt. Dann wird Wasser zugegeben, mit Essigester verdünnt, mit Wasser neutralgewaschen, über Natriumsulfat getrocknet und im Vakuum zur Trockne eingeengt. Man erhält 16 g rohes 4-[1-Hydroxycyclohept-1-yl-1-(imidazolyl)-methyl]-benzonitril.
10 g rohes 4-[1-Hydroxycyclohept-1-yl-1-(imidazolyl)-methyl]-benzonitril werden bei 0° in 100 ml Dichlormethan gelöst und mit 10 ml Thionylchlorid 4 h gerührt. Dann wird im Ölpumpenvakuum zur Trockne eingeengt, in 100 ml Dichlormethan aufgenommen und mit 40 ml Triethylamin 18 h bei 25° gerührt. Zur Aufarbeitung wird mit Wasser versetzt, zweimal mit Dichlormethan extrahiert, über Natriumsulfat getrocknet, im Vakuum zur Trockne eingeengt und an Kieselgel mit Hexan/Essigester chromatographiert. Man erhält 2,03 g 4-[1-Cycloheptyliden-1-(imidazolyl)-methyl]-benzonitril als ölige freie Base.

### Beispiel 4

### 4-[2-Adamantyliden-1-(imidazolyl)-methyl]-benzonitril.

5.0 g 4-[1-(Imidazolyl)-methyl]-benzonitril werden in 100 ml Tetrahydrofuran gelöst und bei -50° mit 20 ml 1,5 molarer Lösung von Lithiumdiisopropylamid in Tetrahydrofuran versetzt, 0,5 h bei -60° gerührt, mit 4,3 g 2-Adamantanon versetzt, 1 h nachgerührt und auf 25° erwärmt. Dann wird Wasser zugegeben, mit dreimal mit Essigester extrahiert, mit Wasser neutralgewaschen, über Natriumsulfat getrocknet und im Vakuum zur Trockne eingeengt. Man erhält 6,66 g rohes 4-[2-Hydroxyadamant-2-yl-1-(imidazolyl)-methyl]-benzonitril.
6,66 g rohes 4-[2-Hydroxyadamant-1-yl-1-(imidazolyl)-methyl]-benzonitril werden in 50 ml Dichlormethan bei 0° gelöst und mit 12,7 ml Thionylchlorid 1 h bei 0° gerührt. Dann wird im Vakuum zur Trockne eingeengt, in 50 ml Dichlormethan gelöst, mit 24 ml Triethylamin 2 h gerührt, mit Wasser verdünnt, viermal mit Essigester extrahiert, mit Kochsalzlösung gewaschen, über Natriumsulfat getrocknet, im Vakuum zur Trockne eingeengt und an Kieselgel mit Dichlormethan/Aceton chromatographiert. Man erhält 1,55 g 4-[2-Adamantyliden-1-(imidazolyl)-methyl]-benzonitril als freie Base vom Schmelzpunkt 155°.

### Beispiel 5

### 4-[1-Cyclohexyliden-1-(1,2,4-triazolyl)-methyl]-benzonitril.

### a) 4-[1-(1,2,4-Triazolyl)-methyl]-benzonitril.

23,5 g 1,2,4-Triazol werden in 250 ml N,N-Dimetylformamid unter Kühlung portionsweise mit 14,15 g Natriumhydrid 60% in Öl versetzt und 1 h bei 25° bis zum Ende der Wasserstoffentwicklung gerührt. Zu dieser Lösung werden bei 5° 50 g 4-(Brommethyl)-benzonitril in 2 Portionen unter Eiskühlung zugegeben und 2 h bei 25° gerührt. Zur Aufarbeitung werden unter Eiskühlung 200 ml Wasser zugesetzt, 1 h gerührt, mit Essigester extrahiert, mit Wasser neutral gewaschen, über Natriumsulfat getrocknet, im Vakuum zur Trockne eingeengt und an Kieselgel mit Dichlormethan/Methanol chromatographiert. Man erhält 31,73 g 4-[1-(1,2,4-Triazolyl)-methyl]-benzonitril als farblose Kristalle vom Schmelzpunkt 72-73°.

### b) 4-[1-Cyclohexyliden-1-(1,2,4-triazolyl)-methyl]-benzonitril.

5 g 4-[1-(1,2,4-Triazolyl)-methyl]-benzonitril werden in 100 ml Tetrahydrofuran gelöst und bei -50° mit 20 ml 1,5 molarer Lösung von Lithiumdiisopropylamid in Tetrahydrofuran versetzt, 0,5 h gerührt, mit 2,9 g Cyclohexanon 1 h bei - 70° nachgerührt und auf 25° erwärmt. Dann wird Wasser zugegeben, mit Essigester extrahiert, mit Wasser neutralgewaschen, über Natriumsulfat getrocknet und im Vakuum zur Trockne eingeengt. Man erhält 7,24 g rohes 4-[1-Hydroxycyclohex-1-yl-1-(1,2,4-triazolyl)-methyl]-benzonitril.
7,24 g rohes 4-[1-Hydroxycyclohex-1-yl-1-(1,2,4-triazolyl)-methyl]-benzonitril werden in 50 ml Dichlormethan bei 0° gelöst und mit 22,7 ml Thionylchlorid 1 h bei 0° gerührt. Dann wird im Vakuum zur Trockne eingeengt, in 50 ml Dichlormethan gelöst, mit 30 ml Triethylamin 2 h gerührt, mit Wasser verdünnt, viermal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 1,45 g 4-[1-Cyclohexyliden-1-(1,2,4-triazolyl)-methyl]-benzonitril als freie Base vom Schmelzpunkt 92-93°.

### Beispiel 6

### 4-[1-Cyclopentyliden-1-(1,2,4-triazolyl)-methyl]-benzonitril.

5 g 4-[1-(1,2,4-Triazolyl)-methyl]-benzonitril werden in 100 ml Tetrahydrofuran gelöst und bei -50° mit 20 ml 1,5 molarer Lösung von Lithiumdiisopropylamid in Tetrahydrofuran versetzt, 0,5 h gerührt, mit 2,4 g Cyclopentanon 1 h bei - 70° nachgerührt und auf 25° erwärmt. Dann wird Wasser zugegeben, zweimal mit Essigester extrahiert, mit Wasser neutralgewaschen, über Natriumsulfat getrocknet und im Vakuum zur Trockne eingeengt. Man erhält 7,21 g rohes 4-[1-Hydroxycyclopent-1-yl-1-(1,2,4-triazolyl)-methyl]-benzonitril.
7,21 g rohes 4-[1-Hydroxycyclopent-1-yl-1-(1,2,4-triazolyl)-methyl]-benzonitril werden in 50 ml Dichlormethan bei 0° gelöst und mit 24 ml Thionylchlorid 1 h bei 0° gerührt. Dann wird im Vakuum zur Trockne eingeengt, in 50 ml Dichlormethan gelöst, mit 32 ml Triethylamin 2 h gerührt, mit Wasser verdünnt, viermal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 2,1 g 4-[1-Cyclopentyliden-1-(1,2,4-triazolyl)-methyl]-benzonitril als freie Base vom Schmelzpunkt 72°.

### Beispiel 7

### 4-[1-Cycloheptyliden-1-(1,2,4-triazolyl)-methyl]-benzonitril.

5 g 4-[1-(1,2,4-Triazolyl)-methyl]-benzonitril werden in 100 ml Tetrahydrofuran gelöst und bei -50° mit 19,8 ml 1,5 molarer Lösung von Lithiumdiisopropylamid in Tetrahydrofuran versetzt, 0,5 h gerührt, mit 3,05 g Cycloheptanon 1 h bei - 70° nachgerührt und auf 25° erwärmt. Dann wird Wasser zugegeben, zweimal mit Essigester extrahiert, mit Wasser neutralgewaschen, über Natriumsulfat getrocknet und im Vakuum zur Trockne eingeengt. Man erhält 7,9 g rohes 4-[1-Hydroxycyclohept-1-yl-1-(1,2,4-triazolyl)-methyl]-benzonitril.
7 g rohes 4-[1-Hydroxycyclohept-1-yl-1-(1,2,4-triazolyl)-methyl]-benzonitril werden in 50 ml Dichlormethan bei 0° gelöst und mit 20 ml Thionylchlorid 1 h bei 0° gerührt. Dann wird im Ölpumpenvakuum zur Trockne eingeengt, in 50 ml Dichlormethan gelöst, mit 30 ml Triethylamin 2 h gerührt, mit Wasser verdünnt, dreimal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 1,9 g 4-[1-Cycloheptyliden-1-(1,2,4-triazolyl)-methyl]-benzonitril als freie Base vom Schmelzpunkt 79-80°.

### Beispiel 8

### 4-[2-Adamantyliden-1-(1,2,4-triazolyl)-methyl]-benzonitril.

5 g 4-[1-(1,2,4-Triazolyl)-methyl]-benzonitril werden in 100 ml Tetrahydrofuran gelöst und bei -50° mit 19,8 ml 1,5 molarer Lösung von Lithiumdiisopropylamid in Tetrahydrofuran versetzt, 0,5 h gerührt, mit 4,25 g Adamantanon 1 h bei - 70° nachgerührt und auf 25° erwärmt. Dann wird Wasser zugegeben, zweimal mit Essigester extrahiert, mit Wasser neutralgewaschen, über Natriumsulfat getrocknet und im Vakuum zur Trockne eingeengt. Man erhält 9 g rohes 4-[2-Hydroxyadamant-2-yl-1-(1,2,4-triazolyl)-methyl]-benzonitril.
9 g rohes 4-[2-Hydroxyadamant-2-yl-1-(1,2,4-triazolyl)-methyl]-benzonitril werden in 50 ml Dichlormethan bei 0° gelöst und mit 24 ml Thionylchlorid 1 h bei 0° gerührt. Dann wird im Ölpumpenvakuum zur Trockne eingeengt, in 50 ml Dichlormethan gelöst, mit 32 ml Triethylamin 2 h gerührt, mit Wasser verdünnt, dreimal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 5,3 g 4-[2-Adamantyliden-1-(1,2,4-triazolyl)-methyl]-benzonitril als freie Base vom Schmelzpunkt 130°, die mit Salzsäure ins Hydrochlorid vom Schmelzpunkt 105° überführt wird.

### Beispiel 9

### 4-[1-Cyclohexyliden-1-(1,2,3-triazolyl)-methyl]-benzonitril.

### a) 4-Azidomethylbenzonitril.

50 g 4-Brommethylbenzonitril werden in 350 ml 1,3-Dimethyl-2-imidazolidinon gelöst und bei 25° 2 h mit 32,5 g Natriumazid gerührt. Anschließend wird unter Eiskühlung mit 500 ml Wasser versetzt, zweimal mit Dichlormethan extrahiert, mit Wasser gewaschen, über Natriumsulfat getrocknet, im Vakuum zur Trockne eingeengt und an Kieselgel mit Hexan/Essigester chromatographiert. Man erhält 39 g 4-Azidomethylbenzonitril als farblose Flüssigkeit.

### b) 4-[1-(1,2,3-Triazolyl)-methyl]-benzonitril.

38,9 g 4-Azidomethylbenzonitril werden in 400 ml Toluol mit 42 ml Trimetylsilylacetylen 5 h bei 120° rückfluxiert, mit weiteren 21 ml Trimetylsilylacetylen versetzt und noch einmal 3 h bei 120° rückfluxiert. Dann wird im Vakuum eingeengt und man erhält 63 g rohes 4-[4-Trimethylsilyl-(1,2,3-triazol-1-yl)-methyl]-benzonitril.
Dieses wird mit einer Mischung von 700 ml Eisessig, 380 ml Wasser sowie 110 ml Tetrahydrofuran 2 h rückfluxiert. Zur Aufarbeitung wird in Natriumhydrogencarbonatlösung gegossen, dreimal mit Dichlormethan extrahiert, mit Wasser neutral gewaschen, über Natriumsulfat getrocknet, im Vakuum zur Trockne eingeengt und aus Essigester/Hexan umkristallisiert. Man erhält 32,8 g 4-[1-(1,2,3-Triazolyl)-methyl]-benzonitril als farblose Kristalle vom Schmelzpunkt 83°.

### c) 4-[1-Cyclohexyliden-1-(1,2,3-triazolyl)-methyl]-benzonitril.

5 g 4-[1-(1,2,3-Triazolyl)-methyl]-benzonitril werden in 100 ml Tetrahydrofuran gelöst und bei -50° mit 20 ml 1,5 molarer Lösung von Lithiumdiisopropylamid in Tetrahydrofuran versetzt, 0,5 h gerührt, mit 2,9 g Cyclohexanon 1 h bei - 70° nachgerührt und auf 25° erwärmt. Dann wird Wasser zugegeben, dreimal mit Essigester extrahiert, mit Wasser gewaschen, über Natriumsulfat getrocknet und im Vakuum zur Trockne eingeengt. Man erhält 7,6 g rohes 4-[1-Hydroxycyclohex-1-yl-1-(1,2,3-triazolyl)-methyl]-benzonitril.
7,6 g rohes 4-[1-Hydroxycyclohex-1-yl-1-(1,2,3-triazolyl)-methyl]-benzonitril werden in 50 ml Dichlormethan bei 0° gelöst und mit 22,7 ml Thionylchlorid 1 h bei 0° gerührt. Dann wird im Vakuum zur Trockne eingeengt, in 50 ml Dichlormethan gelöst, mit 30 ml Triethylamin 2 h gerührt, mit Wasser verdünnt, dreimal mit Essigester extrahiert, mit Wasser gewaschen, über Natriumsulfat getrocknet, im Vakuum zur Trockne eingeengt und an Kieselgel mit Hexan/Essigester chromatographiert. Man erhält 2,6 g 4-[1-Cyclohexyliden-1-(1,2,3-triazolyl)-methyl]-benzonitril als freie Base, Schmelzpunkt 132-133°C.

### Beispiel 10

### 4-[1-Cyclopentyliden-1-(1,2,3-triazolyl)-methyl]-benzonitril.

5 g 4-[1-(1,2,3-Triazolyl)-methyl]-benzonitril werden in 100 ml Tetrahydrofuran gelöst und bei -60° mit 20 ml 1,5 molarer Lösung von Lithiumdiisopropylamid in Tetrahydrofuran versetzt, 0,5 h gerührt, mit 2,4 g Cyclopentanon 1 h bei - 70° nachgerührt und auf 25° erwärmt. Dann wird Wasser zugegeben, dreimal mit Essigester extrahiert, mit Wasser gewaschen, über Natriumsulfat getrocknet und im Vakuum zur Trockne eingeengt. Man erhält 7,1 g rohes 4-[1-Hydroxycyclopent-1-yl-1-(1,2,3-triazolyl)-methyl]-benzonitril.
7,1 g rohes 4-[1-Hydroxycyclopent-1-yl-1-(1,2,3-triazolyl)-methyl]-benzonitril werden in 50 ml Dichlormethan bei 0° gelöst und mit 24 ml Thionylchlorid 1 h bei 0° gerührt. Dann wird im Vakuum zur Trockne eingeengt, in 50 ml Dichlormethan gelöst, mit 32 ml Triethylamin 2 h gerührt, mit Wasser verdünnt, dreimal mit Essigester extrahiert, mit Wasser gewaschen, über Natriumsulfat getrocknet, im Vakuum zur Trockne eingeengt und an Kieselgel mit Hexan/Essigester chromatographiert. Man erhält 2,1 g 4-[1-Cyclopentyliden-1-(1,2,3-triazolyl)-methyl]-benzonitril als freie Base vom Schmelzpunkt 108-109°C.

### Beispiel 11

### 5-(Cyclohexyliden-1-imidazolylmethyl)-thiophen-2-carbonitril.

### a) 1-(5-Brom-2-thienylmethyl)-imidazol.

20 g 5-Bromthiophen-2-carbaldehyd werden mit 4 g Natriumborhydrid in 100 ml 2-Propanol in üblicher Weise zu 20 g 5-Brom-2- thiophenmethanol reduziert. Dieses wird mit 30 ml Dichlormethan gelöst und mit 25 ml Thioylchlorid bei 25° 2 h gerührt. Nach Eindampfen im Vakuum und zweimaligem Nachdestillieren mit Toluol wird der Rückstand in 15 ml Dimethylformamid gelöst und zu einer aus 7,14 g Imidazol und 3,15 g Natriumhydrid-Ölsuspension (85%) in 30 ml Dimethylformamid bereiteten Lösung von Imidazolnatrium zugetropft. Nach 20 h bei 25° wird das Gemisch auf 2 N Salzsäure gegeben und mit Ether extrahiert. Die wässrige Pase wird mit Kaliumcarbonat alkalisiert und mit Essigester extrahiert, die organische Phase mit Wasser gewaschen, über Natriumsulfat getrocknet, am Vakuum eingeengt und am Kugelrohr destilliert. Man erhält 17,3 g 1-(5-Brom-2-thienylmethyl)-imidazol vom Siedepunkt 170° bei 0,03 mbar.

### b) 5-(1-Imidazolylmethyl)-thiophen-2-carbonitril.

10 g 1-(5-Brom-2-thienylmethyl)-imidazol werden mit 10 g Kupfer(I)cyanid in 150 ml Dimethylformamid 20 h rückfluxiert. Dann wird das Gemisch auf Wasser gegeben, die Kristalle werden abgesaugt und mit 200 ml 25%igem Ammonikwasser eine Stunde bei Raumtemperatur gerührt. Dann wird mit Essigester extrahiert, mit Wasser gewaschen, über Natriumsulfat getrocknet, am Vakuum eingeengt und am Kugelrohr destilliert. Man erhält 6,2 g 5-(1-Imidazolylmethyl)-thiophen-2-carbonitril als orange Kristalle vom Schmelzpunkt 73-75°.

### c) 5-(Cyclohexyliden-1-imidazolylmethyl)-thiophen-2-carbonitril.

1,8 g 5-(1-Imidazolylmethyl)-thiophen-2-carbonitril werden in 30 ml Tetrahydrofuran bei -50° mit 7,3 mi einer 1,5 molaren Lösung von Lithiumdiisopropylamid in Tetrahydrofuran versetzt und 0,5 h nachgerührt. Anschließend werden 1,05 g Cyclohexanon bei -70° zugetropft, 1 h bei -70° gerührt und dann langsam auf 25° erwärmt. Zur Aufarbeitung gibt man das Reaktionsgemisch auf Wasser, extrahiert mit Essigester, extrahiert die organische Phase mit 2 normaler Salzsäure, stellt mit Kaliumcarbonat alkalisch, extrahiert erneut mit Essigester, trocknet über Natriumsulfat und engt im Vakuum zur Trockne ein. Man erhält rohes 5-[Hydroxy-(1-imidazoyl)-methyl]-thiophen-2-carbonitril als Schaum.
Dieser Schaum wird in 10 ml Dichlormethan bei 0° mit 5 ml Thionylchlorid 1 h gerührt, im Ölpumpenvakuum zur Trockne eingeengt und mit 7 ml Triethylamin 20 h bei 25° gerührt. Dann wird auf Wasser gegeben, mit Essigester extrahiert, mit Wasser gewaschen,über Natriumsulfat getrocknet, in Vakuum eingeengt und an Kieselgel mit Dichlormethan/Isopropanol chromatographiert. Man erhält 180 mg 5-(Cyclohexyliden-1-imidazolylmethyl)-thiophen-2-carbonitril als gelbes Öl neben etwas 5-[(1-Cyclohexenyl)-(1-imidazolyl)-methyl]-thiophen-2-carbonitril.

### 5-[Cyclopentyliden-(1-imidazolyl)-methyl]-thiophen-2-carbonitril

a. Aus 1,8 g 5-(1-Imidazolylmethyl)-thiophen-2-carbonitril und 0.93 ml Cyclopentanon erhält man analog Beispiel 11 ein Rohprodukt, das am Kugelrohr bei 230°/0.03 mbar destilliert wird. Nach Kristallisation aus Ether erhält man aus dem Destillat 120 mg der Titelverbindung als farblose Kristalle vom Schmelzpunkt 90-92°C.
b.
   1) 5-Cyclopentylidenmethyl-thiophen-2-carbonitril
      8 g 5-Brommethyl-thiophen-2-carbonitril (hergestellt durch Bromierung von 5,7 g 5-Methyl-thiophen-2-carbonitril und 8,42 g N-Bromsuccinimid in 68 ml Tetrachlorkohlenstoff unter Belichtung und Zusatz von 185 mg Benzoylperoxid) werden in 35 ml Chloroform mit 5,6 g Hexamethylentetramin 30 Minuten am Rückfluß gekocht. Das ausgefallene Salz wird abgesaugt und in 30 ml Essigsäure und 30 ml Wasser 2 Stunden am Rückfluß gekocht. Nach Extraktion mit Ether, Waschen der Etherphase mit Wasser, Trocknen und Eindampfen erhält man 2.43 g 5-Cyanthiophen-2-aldehyd.
      Aus 3 g Cyclohexyl-triphenylphosphoniumbromid in 80 ml Ether stellt man durch Zutropfen von 4,5 ml einer 1,5 molaren Lösung von Butyllithium in Hexan bei Raumtemperatur das entsprechende Phosphoran her, zu dem 1 g 5-Cyanthiophen-2-aldehyd in 45 ml Ether zugetropft werden. Nach 16 Stunden Rühren bei Raumtemperatur wird ein Niederschlag abgesaugt und das Filtrat eingedampft. Der Rückstand wird aus Isopropanol umkristallisiert. Man erhält 610 mg der Titelverbindung b. 1) vom Schmelzpunkt 98-100°C.
      In einem zweiten Verfahren werden 20 g 5-Brommethyl-thiophen-2-carbonitril mit 200 ml Triethylphosphit 4 Stunden auf 150°C erwärmt. Danach wird bei 40-60°C/0,06 mbar Triethylphosphit entfernt und der Rückstand bei 130-150°C/0,004 mbar am Kugelrohr destilliert. Man erhält so 24 g (5-Cyan-2-thienyl)-methyl-phosphonsäurediethylester. 5 g davon werden mit 1,5 ml Cyclopentanon in 5 ml Tetrahydrofuran zu einer Suspension von 581 mg Natriumhydrid/Öl (80%ig) und 116 mg 15-Krone-5 in 5 ml Tetrahydrofuran getropft.
      Nach 2 Stunden Rühren bei Raumtemperatur wird auf Wasser gegeben und mit Essigester extrahiert. Die Essigesterphase wird getrocknet und eingedampft und der Rückstand aus Isopropanol umkristallisiert. Man erhält 3,0 g 5-Cyclopentylidenmethyl-thiophen-2-carbonitril, identisch mit dem oben beschriebenen Produkt.
   2) 3 g 5-Cyclopentylidenmethyl-thiophen-2-carbonitril werden in 30 ml Dichlormethan gelöst und mit 31 ml einer 0,5 molaren Lösung von Brom in Dichlormethan versetzt. Nach 3 Stunden Rühren bei Raumtemperatur wird mit Essigester verdünnt, mit verdünnter Natriumthiosulfatlösung geschüttelt, getrocknet und eingedampft. Der Rückstand wird mit 13 g Imidazol 12 Stunden unter Argon bei 120°C gehalten, dann auf 2 molare Salzsäure gegeben und mit Ether extrahiert. Die Wasserphase wird mit Kaliumcarbonbat alkalisiert und mit Essigester extrahiert. Nach Waschen der Essigesterphase mit Wasser, Trocknen und Eindampfen erhält man ein Rohprodukt, das über 250 g Kieselgel mit Dichlormethan/Isopropanol (100:0 bis 97:3) chromatographiert wird. Danach wird aus Ether kristallisiert und man erhält so 70 mg 5-[Cyclopentyliden-(1-imidazolyl)-methyl]-thiophen-2-carbonitril, identisch mit dem unter a. beschriebenen Material.

Analog zu den vorstehenden Vorschriften wurden weitere erfindungsgemäße Verbindungen der allgemeinen Formel I hergestellt, nämlich:
4[1-(3,3,5,5,-Tetramethyl-1-cyclohexyliden)-1-(1-imidazolyl)-methyl]-benzonitril; Fp.: 111-113°C
4-[1-Cycloheptyliden-1-(1,2,3-triazol-1-yl)-methyl]-benzonitril; Fp.: 91-93°C
4-[1-Cyclodecyliden-1-(1-imidazolyl)-methyl]-benzonitril; Öl
4-[1-Cycloheptyliden-1-(1-imidazolyl)-methyl]-benzonitril, Hydrochlorid; Öl
4-[1-(4-tert.-Butyl-1-cyclohexyliden)-1-(1-imidazolyl)-methyl]-benzonitril; Öl
4-[1-(3,4-Dimethyl-1-cyclopentyliden)-1-(1-imidazolyl)-methyl]-benzonitril;
(Isomerengemisch cis/trans); Öl 4-[1-(3,5-Dimethyl-1-cyclohexyliden)-1-(1-imidazolyl)-methyl]-benzonitril; Öl
4-[1-(4-Methyl-1-cyclohexyliden)-1-(1-imidazolyl)-methyl]-benzonitril; Fp.:85-87°C
4-[1-Cyclononyliden)-1-(1-imidazolyl)-methyl]-benzonitril; Öl 4-[1-(Bicyclo[3,3,1]-non-9-yliden)-1-(1-imidazolyl)-methyl]-benzonitril; Fp.: 138-139°C
4-[1-(1-Imidazolyl)-1-(spiro[5,5]undec-3-yliden)-methyl]-benzonitril; Öl
4-[1-Cyclooctyliden)-1-(1-imidazolyl)-methyl]-benzonitril; Fp.: 111-112°C
4-[1-(2,6-Dimethyl-1-cyclohexyliden)1-1(1-imidazolyl)-methyl]-benzonitril; Öl
4-[1-Cyclohexyliden-1-(1-imidazolyl)-methyl]-benzonitril; Fp.: 128°C
4-[1-(2-Adamantyliden)-1-(1,2,3-triazol-1-yl)-methyl]-benzonitril; Fp.:151-153°C
4-[1-(2-Adamantyliden)-1-(1,2,4-triazol-1-yl)-methyl]-benzonitril; Fp.: 105°C
4-[1-(2-Adamantyliden)-1-(1-imidazolyl)-methyl]-benzonitril, Hydrochlorid; Schaum
4-[1-Cyclobutyliden)-1-(1-imidazolyl)-methyl]-benzonitril; Fp.: 120-122°C

## Patentansprüche

1. Cycloalkylenazole der allgemeinen Formel I 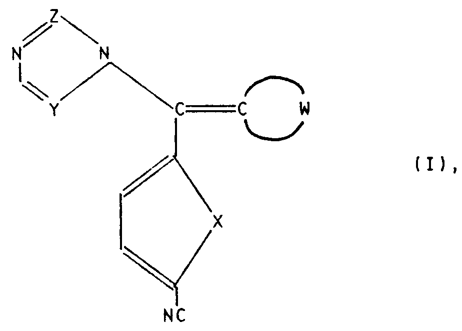 worin 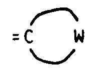 für ein carbocyclisches oder carbopolycyclisches System, das gegebenenfalls mindestens einen Alkylsubstituenten trägt,
X für die Gruppierung 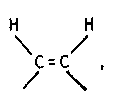 für ein Sauerstoff- oder Schwefelatom und
Y und Z unabhängig voneinander für eine Methingruppe 〉̶CH oder ein Stickstoff atom
stehen, sowie deren pharmazeutisch verträgliche Additionssalze mit Säuren.

2. Cycloalkylenazole nach Anspruch 1, dadurch gekennzeichnet, daß 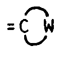 für einen Cyclopentyliden-, Cyclohexyliden- oder Cycloheptylidenrest steht.

3. Cycloalkylenazole nach Anspruch 1, dadurch gekennzeichnet, daß 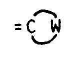 für einen Cyclobutyliden-, Cyclooctyliden-, Cyclononyliden-, Cyclodecylidenrest steht.

4. Cycloalkylenazole nach Anspruch 1, dadurch gekennzeichnet, daß 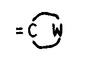 für einen 2-Adamantylidenrest steht.

5. Cycloalkylenazole nach Anspruch 1, dadurch gekennzeichnet, daß 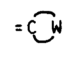 für einen Bicyclo[3,3,1]-non-9-yliden- oder Spiro[5,5]undec-3-ylidenrest steht.

6. Cycloalkylenazole nach Anspruch 1, dadurch gekennzeichnet, daß 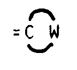 mindestens einen geradkettigen oder verzweigten C₁- bis C₆-Alkylsubstituenten trägt.

7. Cycloalkylenazole nach Anspruch 1, dadurch gekennzeichnet, daß 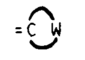 ein carbocyclisches System ist und 1 bis 4 geradkettige oder verzweigte C₁- bis C₄-Alkylsubstituenten trägt.

8. Cycloalkenylazole nach Anspruch 1, dadurch gekennzeichnet, daß X für eine 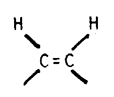 - Brücke steht.

9. Cycloalkenylazole nach Anspruch 1, dadurch gekennzeichnet, daß X für ein Schwefelatom steht.

10. Cycloalkylenazole nach Anspruch 1, dadurch gekennzeichnet, daß 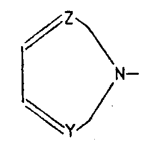 für einen 1-Imidazolyl-, 1,2,3- oder 1,2,4-Triazolyl-ring steht.

11. 4-[1-Cyclohexyliden-1-(imidazolyl)-methyl]-benzonitril, Hydrochlorid;
4-[1-Cyclopentyliden-1-(imidazolyl)-methyl]-benzonitril;
4-[1-Cycloheptyliden-1-(imidazolyl)-methyl]-benzonitril;
4-[2-Adamantyliden-1-(imidazolyl)-methyl]-benzonitril;
4-[1-Cyclohexyliden-1-(1,2,4-triazolyl)-methyl]-benzonitril;
4-[1-Cyclopentyliden-1-(1,2,4-triazolyl)-methyl]-benzonitril;
4-[1-Cycloheptyliden-1-(1,2,4-triazolyl)-methyl]-benzonitril;
4-[2-Adamantyliden-1-(1,2,4-triazolyl)-methyl]-benzonitril;
4-[1-Cyclohexyliden-1-(1,2,3-triazolyl)-methyl]-benzonitril;
4-[1-Cyclopentyliden-1-(1,2,3-triazolyl)-methyl]-benzonitril;
5-(Cyclohexyliden-1-imidazolylmethyl]-thiophen-2-carbonitril.
4[1-(3,3,5,5-Tetramethyl-1-cyclohexyliden)-1-(1-imidazolyl)-methyl]-benzonitril;
4-[1-Cycloheptyliden-1-(1,2,3-triazol-1-yl)-methyl]-benzonitril;
4-[1-Cyclodecyliden-1-(1-imidazolyl)-methyl]-benzonitril;
4-[1-Cycloheptyliden-1-(1-imidazolyl)-methyl]-benzonitril, Hydrochlorid;
4-[1-(4-tert.-Butyl-1-cyclohexyliden)-1-(1-imidazolyl)-methyl]-benzonitril;
5-[Cyclopentyliden-(1-imidazolyl)-methyl]-thiophen-2-carbonitril
4-[1-(3,4-Dimethyl-1-cyclopentyliden)-1-(1-imidazolyl)-methyl]-benzonitril; (Isomerengemisch cis/trans);
4-[1-(3,5-Dimethyl-1-cyclohexyliden)-1-(1-imidazolyl)-methyl]-benzonitril;
4-[1-(4-Methyl-1-cyclohexyliden)-1-(1-imidazolyl)-methyl]-benzonitril;
4-[1-Cyclononyliden)-1-(1-imidazolyl)-methyl]-benzonitril;
4-[1-(Bicyclo[3,3,1]-non-9-yliden)-1-(1-imidazolyl)-methyl]-benzonitril;
4-[1-(1-Imidazolyl)-1-(spiro[5,5]undec-3-yliden)-methyl]-benzonitril;
4-[1-Cyclooctyliden)-1-(1-imidazolyl)-methyl]-benzonitril;
4-[1-(2,6-Dimethyl-1-cyclohexyliden)-1-(1-imidazolyl)-methyl]-benzonitril;
4-[1-Cyclohexyliden-1-(1-imidazolyl)-methyl]-benzonitril;
4-[1-(2-Adamantyliden)-1-(1,2,3-triazol-1-yl)-methyl]-benzonitril;
4-[1-(2-Adamantyliden)-1-(1,2,4-triazol-1-yl)-methyl]-benzonitril;
4-[1-(2-Adamantyliden)-1-(1-imidazolyl)-methyl]-benzonitril, Hydrochlorid;
4-[1-Cyclobutyliden)-1-(1-imidazolyl)-methyl]-benzonitril;

12. Verfahren zur Herstellung von Cycloalkylenazolen der allgemeinen Formel I 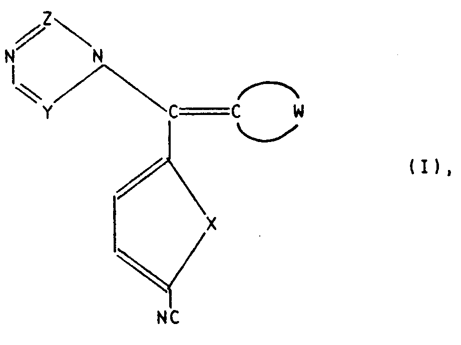 worin 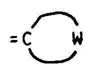 für ein carbocyclisches oder carbopolycyclisches System, das gegebenenfalls mindestens einen Alkylsubstituenten trägt,
X für die Gruppierung 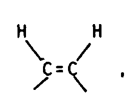 für ein Sauerstoff- oder Schwefelatom und
Y und Z unabhängig voneinander für eine Methingruppe 〉̶CH oder ein Stickstoffatom
stehen,
dadurch gekennzeichnet, daß eine Verbindung der allgemeinen Formel II 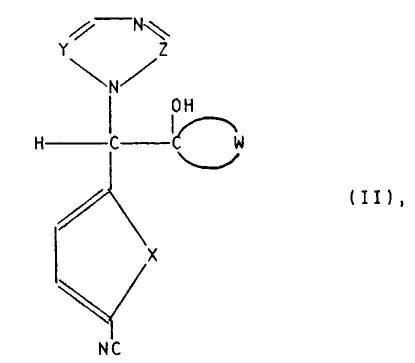 worin 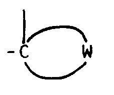 für ein carbocyclisches oder carbopolycyclisches System steht sowie
X, Y und Z die in Formel I angegebene Bedeutung haben,
gegebenenfalls nach Überführung der Hydroxygruppe in eine bessere Abgangsgruppe A, entweder durch Erhitzen oder durch Zugabe einer Base, gegebenenfalls unter zusätzlichem Erhitzen, durch Abspaltung von HOH oder, falls die Hydroxygruppe in eine Abgangsgruppe A überführt wurde, durch Abspaltung von HA oder
daß ein Dihalogenid der allgemeinen Formel III 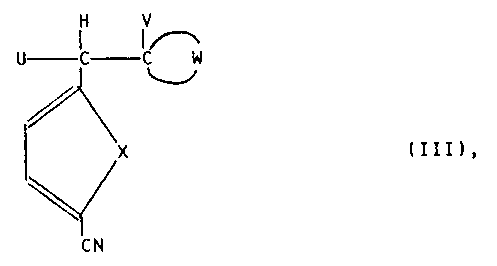 worin 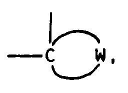 X, Y und Z die in Formel I angegebene Bedeutung haben und U und V Halogenatome bedeuten, die gleich oder verschieden, vorzugsweise 2 Bromatome, sein können, durch Umsetzung mit einem Heterocyclus der allgemeinen Formel IV 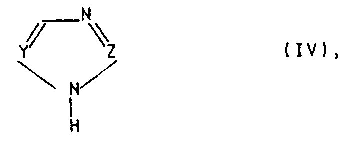 worin
Y und Z die in Formel I angegebene Bedeutung haben, gegebenenfalls unter Verwendung einer Hilfsbase, in eine Verbindung der allgemeinen Formel I und diese gegebenenfalls durch Umsetzung mit Säure in ein pharmazeutisch verträgliches Additionssalz überführt wird.

13. Pharmazeutische Präparate, dadurch gekennzeichnet, daß sie mindestens eine Verbindung der allgemeinen Formel I sowie einen pharmazeutisch verträglichen Träger enthalten.

14. Verwendung von Verbindungen der allgemeinen Formel I zur Herstellung von Arzneimitteln.

## Claims

1. Cycloalkyleneazoles of the general formula I 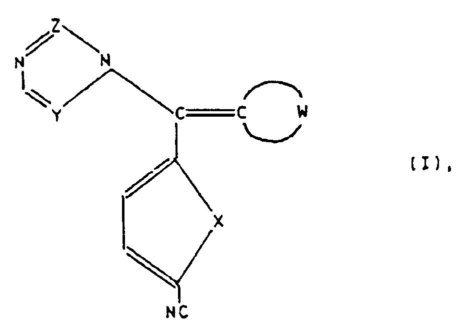 wherein 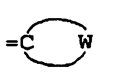 is a carbocyclic or carbopolycyclic system which optionally carries at least one alkyl substituent,
X is the grouping 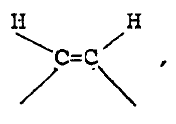 or an oxygen or sulfur atom and
Y and Z are each independently of the other a methine group 〉̶CH or a nitrogen atom,
and the pharmaceutically acceptable addition salts thereof with acids.

2. Cycloalkyleneazoles according to claim 1, characterised in that 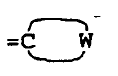 is a cyclopentylidene, cyclohexylidene or cycloheptylidene radical.

3. Cycloalkyleneazoles according to claim 1, characterised in that 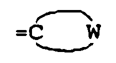 is a cyclobutylidene, cyclooctylidene, cyclononylidene or cyclodecylidene radical.

4. Cycloalkyleneazoles according to claim 1, characterised in that 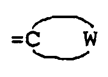 is a 2-adamantylidene radical.

5. Cycloalkyleneazoles according to claim 1, characterised in that 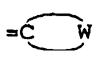 is a bicyclo[3,3,1]non-9-ylidene or spiro[5,5]-undec-3-ylidene radical.

6. Cycloalkyleneazoles according to claim 1, characterised in that 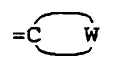 carries at least one straight-chained or branched C₁- to C₆-alkyl substituent.

7. Cycloalkyleneazoles according to claim 1, characterised in that 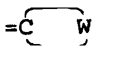 is a carbocyclic system and carries from 1 to 4 straight-chained or branched C₁- to C₄-alkyl substituents.

8. Cycloalkyleneazoles according to claim 1, characterised in that X is a 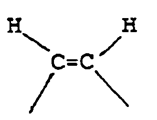 bridge.

9. Cycloalkyleneazoles according to claim 1, characterised in that X is a sulfur atom.

10. Cycloalkyleneazoles according to claim 1, characterised in that 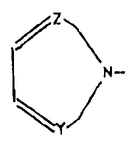 is a 1-imidazolyl, 1,2,3- or 1,2,4-triazolyl ring.

11. 4-[1-Cyclohexylidene-1-(imidazolyl)-methyl]-benzonitrile, hydrochloride;
4-[1-cyclopentylidene-1-(imidazolyl)-methyl-benzonitrile;
4-[1-cycloheptylidene-1-(imidazolyl)-methyl]-benzonitrile;
4-[2-adamantylidene-1-(imidazolyl)-methyl]-benzonitrile;
4-[1-cyclohexylidene-1-(1,2,4-triazolyl)-methyl]-benzonitrile;
4-[1-cyclopentylidene-1-(1,2,4-triazolyl)-methyl]-benzonitrile;
4-[1-cycloheptylidene-1-(1,2,4-triazolyl)-methyl]-benzonitrile;
4-[2-adamantylidene-1-(1,2,4-triazolyl)-methyl]-benzonitrile;
4-[1-cyclohexylidene-1-(1,2,3-triazolyl)-methyl]-benzonitrile;
4-[1-cyclopentylidene-1-(1,2,3-triazolyl)-methyl]-benzonitrile;
5-(cyclohexylidene-1-imidazolylmethyl]-thiopene-2-carbonitrile;
4-[1-(3,3,5,5-tetramethyl-1-cyclohexylidene)-1-(1-imidazolyl)-methyl]-benzonitrile;
4-[1-cycloheptylidene-1-(1,2,3-triazol-1-yl)-methyl]-benzonitrile;
4-[1-cyclodecylidene-1-(1-imidazolyl)-methyl]-benzonitrile;
4-[1-cycloheptylidene-1-(1-imidazolyl)-methyl]-benzonitrile, hydrochloride;
4-[1-(4-tert.-butyl-1-cyclohexylidene)-1-(1-imidazolyl)-methyl]-benzonitrile;
5-[cyclopentylidene-(1-imidazolyl)-methyl]-thiophene-2-carbonitrile;
4-[1-(3,4-dimethyl-1-cyclopentylidene)-1-(1-imidazolyl)-methyl]-benzonitrile; (cis/trans isomeric mixture);
4-[1-(3,5-dimethyl-1-cyclohexylidene)-1-(1-imidazolyl)-methyl]-benzonitrile;
4-[1-(4-methyl-1-cyclohexylidene)-1-(1-imidazolyl)-methyl]-benzonitrile;
4-[1-cyclononylidene)-1-(1-imidazolyl)-methyl]-benzonitrile;
4-[(bicyclo[3.3.1]non-9-ylidene)-1-(1-imidazolyl)-methyl]-benzonitrile;
4-[1-(1-imidazolyl-1-(spiro[5.5]undec-3-ylidene)-methyl]-benzonitrile;
4-[1-cyclooctylidene)-1-(1-imidazolyl)-methyl]-benzonitrile;
4-[1-(2,6-dimethyl-1-cyclohexylidene)-1-(1-imidazolyl)-methyl]-benzonitrile;
4-[1-cyclohexylidene-1-(1-imidazolyl)-methyl]-benzonitrile;
4-[1-(2-adamantylidene)-1-(1,2,3-triazol-1-yl)-methyl-benzonitrile;
4-[1-(2-adamantylidene)-1-(1,2,4-triazol-1-yl)-methyl)-benzonitrile;
4-[1-(2-adamantylidene)-1-(1-imidazolyl)-methyl]-benzonitrile, hydrochloride;
4-[1-cyclobutylidene)-1-(1-imidazolyl)-methyl]-benzonitrile.

12. Process for the preparation of cycloalkyleneazoles of the general formula I 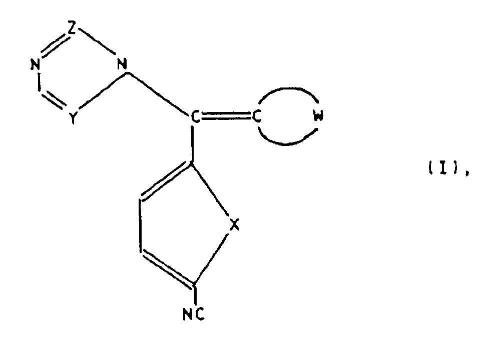 wherein 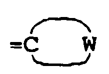 is a carbocyclic or carbopolycyclic system that optionally carries at least one alkyl substituent,
X is the grouping 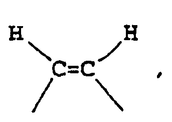 an oxygen or sulfur atom and
Y and Z are each independently of the other a methine group 〉̶CH or a nitrogen atom,
characterised in that a compound of the general formula II 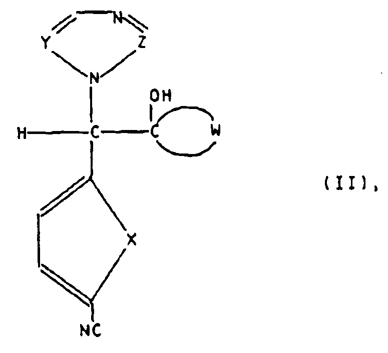 wherein 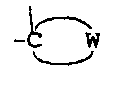 is a carbocyclic or carbopolycyclic system and
X, Y and Z have the meaning given for formula I,
if desired after conversion of the hydroxy group into a better leaving group A, either by heating or by the addition of a base, if necessary with additional heating, by removal of HOH or, if the hydroxy group has been converted into a leaving group A, by removal of HA, or
a dihalide of the general formula III 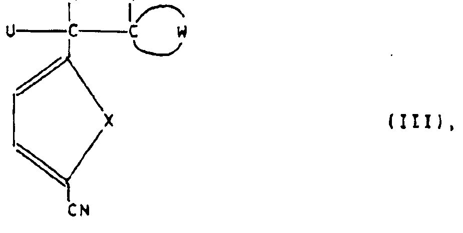 wherein 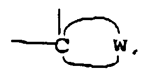 X, Y and Z have the meaning given for formula I and U and V are halogen atoms, which may be identical or different, preferably two bromine atoms, by reaction with a heterocycle of the general formula IV 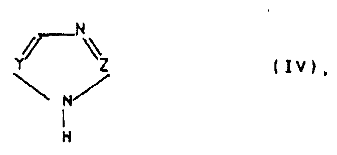 wherein Y and Z have the meaning given for formula I, if necessary with the use of an auxiliary base,
is converted into a compound of the general formula I and the latter is optionally converted by reaction with an acid into a pharmaceutically acceptable addition salt.

13. Pharmaceutical preparations, characterised in that they contain at least one compound of the general formula I and a pharmaceutically acceptable carrier.

14. Use of compounds of the general formula I for the preparation of medicaments.

## Revendications

1. Cycloalkylène-azoles répondant à la formule générale I 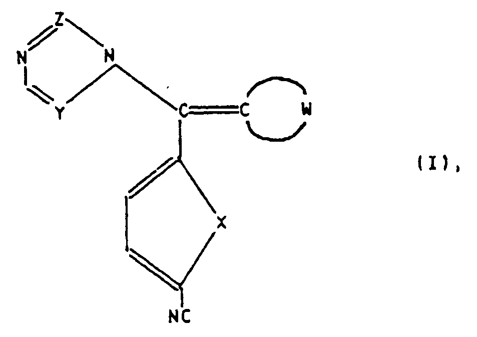 dans laquelle 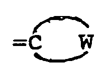
représente un système carbocyclique ou carbopolycyclique, qui porte éventuellement au moins un substituant alkyle,
X représente le groupement 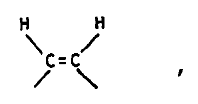 ou un atome d'oxygène ou de soufre, et
Y et Z représentent indépendamment l'un de l'autre un groupe méthyne 〉̶CH ou un atome d'azote,
ainsi que leurs sels d'addition avec des acides, pharmaceutiquement compatibles.

2. Cycloalkylène-azoles suivant la revendication 1, caractérisés en ce que 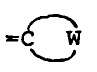 représente un radical cyclopentylidène, cyclohexylidène ou cycloheptylidène.

3. Cycloalkylène-azoles suivant la revendication 1, caractérisés en ce que 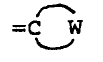 représente un radical cyclobutylidène, cyclooctylidène, cyclononylidène, cyclodécylidène.

4. Cycloalkylène-azoles suivant la revendication 1, caractérisés en ce que 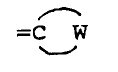 représente un radical 2-adamantylidène.

5. Cycloalkylène-azoles suivant la revendication 1, caractérisés en ce que 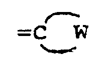 représente un radical bicyclo[3,3,1]-non-9-ylidène ou spiro-[5,5]-undéc-3-ylidène.

6. Cycloalkylène-azoles suivant la revendication 1, caractérisés en ce que 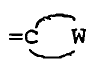 porte au moins un substituant alkyle en C₁-C₆ linéaire ou ramifié.

7. Cycloalkylène-azoles suivant la revendication 1, caractérisés en ce que 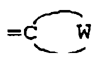 est un système carbocyclique et porte 1 à 4 substituants alkyle en C₁-C₄ linéaires ou ramifiés.

8. Cycloalkylène-azoles suivant la revendication 1, caractérisés en ce que X représente un pont

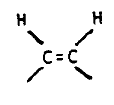

9. Cycloalkylène-azoles suivant la revendication 1, caractérisés en ce que X représente un atome de soufre.

10. Cycloalkylène-azoles suivant la revendication 1, caractérisés en ce que 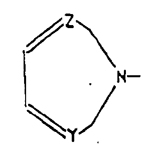 représente un noyau 1-imidazolyle, 1,2,3-triazolyle ou 1,2,4-triazolyle.

11. Chlorhydrate de 4-[1-cyclohexylidène-1-(imidazolyl)-méthyl]-benzonitrile ;
4-[1-cyclopentylidène-1-(imidazolyl)-méthyl]-benzonitrile ;
4-[1-cycloheptylidène-1-(imidazolyl)-méthyl]-benzonitrile ;
4-[2-adamantylidène-1-(imidazolyl)-méthyl]-benzonitrile ;
4-[1-cyclohexylidène-1-(1,2,4-triazolyl)-méthyl]-benzonitrile ;
4-[1-cyclopentylidène-1-(1,2,4-triazolyl)-méthyl]-benzonitrile ;
4-[1-cycloheptylidène-1-(1,2,4-triazolyl)-méthyl]-benzonitrile ;
4-[2-adamantylidène-1-(1,2,4-triazolyl)-méthyl]-benzonitrile ;
4-[1-cyclohexylidène-1-(1,2,3-triazolyl)-méthyl]-benzonitrile ;
4-[1-cyclopentylidène-1-(1,2,3-triazolyl)-méthyl]-benzonitrile ;
5-[cyclohexylidène-1-imidazolylméthyl]-thiophène-2-cabonitrile ;
4-[1-(3,3,5,5-tétraméthyl-1-cyclohexylidène)-1-(1-imidazolyl)-méthyl]-benzonitrile ;
4-[1-cycloheptylidène-1-(1,2,3-triazol-1-yl)-méthyl]-benzonitrile ;
4-[1-cyclodécylidène-1-(imidazolyl)-méthyl]-benzonitrile ;
Chlorhydrate de 4-[1-cycloheptylidène-1-(1-imidazolyl)-méthyl]-benzonitrile ;
4-[1-(4-tert.-butyl-1-cyclohexylidène)-1-(1-imidazolyl)-méthyl]-benzonitrile ;
5-[cyclopentylidène-(1-imidazolyl)-méthyl]-thiophène-2-carbonitrile ;
4-[1-(3,4-diméthyl-1-cyclopentylidène)-1-(1-imidazolyl)-méthyl]-benzonitrile, (mélange d'isomères cis/trans) ;
4-[1-(3,5-diméthyl-1-cyclohexylidène)-1-(1-imidazolyl)-méthyl]-benzonitrile ;
4-[1-(4-méthyl-1-cyclohexylidène)-1-(1-imidazolyl)-méthyl]-benzonitrile ;
4-[1-cyclononylidène)-1-(1-imidazolyl)-méthyl]-benzonitrile ;
4-[1-(bicyclo[3,3,1]-non-9-ylidène)-1-(1-imidazolyl)-méthyl]-benzonitrile ;
4-[1-(1-imidazolyl)-1-(spiro[5,5]undéc-3-ylidène)-méthyl]-benzonitrile ;
4-[1-cyclooctylidène)-1-(1-imidazolyl)-méthyl]-benzonitrile ;
4-[1-(2,6-diméthyl-1-cyclohexylidène)-1-(1-imidazolyl)-méthyl]-benzonitrile ;
4-[1-cyclohexylidène-1-(1-imidazolyl)-méthyl]-benzonitrile ;
4-[1-(2-adamantylidène)-1-(1,2,3-triazol-1-yl)-méthyl]-benzonitrile ;
4-[1-(2-adamantylidène)-1-(1,2,4-triazol-1-yl)-méthyl]-benzonitrile ;
chlorhydrate de 4-[1-(2-adamantylidène)-1-(1-imidazolyl)-méthyl]-benzonitrile ;
4-[1-cyclobutylidène-1-(1-imidazolyl)-méthyl]-benzonitrile.

12. Procédé de préparation de cycloalkylène-azoles répondant à la formule générale I 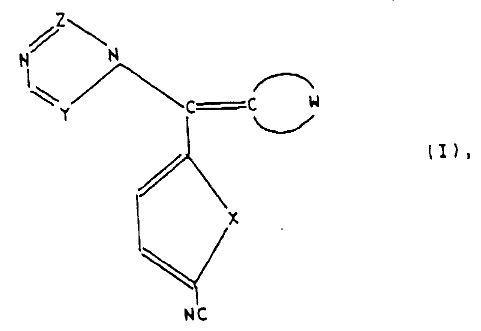 dans laquelle 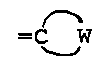 représente un système carbocyclique ou carbopolycyclique, qui porte éventuellement au moins un substituant alkyle,
X représente le groupement 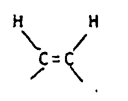 ou un atome d'oxygène ou de soufre et
X et Z représentent indépendamment l'un de l'autre un groupe méthyne 〉̶CH ou un atome d'azote,
caractérisé en qu'on convertit un composé de la formule générale II 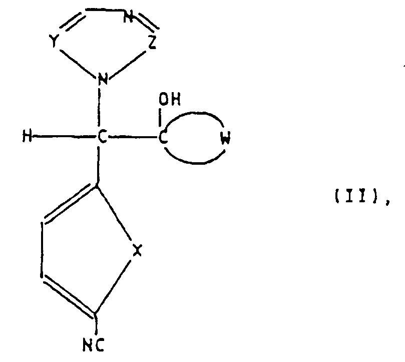 dans laquelle 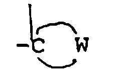 représente un système carbocyclique ou carbopolycyclique et X, Y et Z ont la signification indiquée dans la formule I,
éventuellement après conversion du groupe hydroxy en un meilleur groupe de départ A, par chauffage ou par addition d'une base, éventuellement avec chauffage additionnel, par séparation de HOH ou, dans le cas où le groupe hydroxy a été converti en un groupe de départ A, par séparation de HA, ou
un dihalogénure de la formule générale III 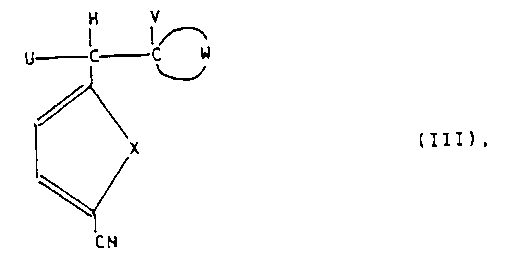 dans laquelle 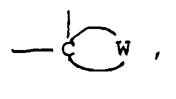 X, Y et Z ont la même signification que celle donnée dans la formule I et U et V représentent des atomes d'halogène qui peuvent être identiques ou différents, de préférence 2 atomes de brome, par réaction avec un hétérocycle de la formule générale IV 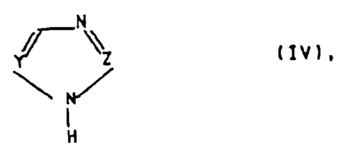 dans laquelle
Y et Z ont la signification indiquée dans la formule I, éventuellement avec utilisation d'une base auxiliaire, en un composé de la formule générale I et en ce qu'on convertit éventuellement ce dernier par réaction avec un acide en un sel d'addition pharmaceutiquement compatible.

13. Préparations pharmaceutiques, caractérisées en ce qu'elles contiennent au moins un composé de la formule générale I ainsi qu'un support pharmaceutiquement compatible.

14. Utilisation de composés suivant la formule générale I, pour la fabrication de médicaments.
